(19) Europäisches Patentamt
European Patent Office
Office européen des brevets

(11) EP 1 653 948 B1

(12) **EUROPEAN PATENT SPECIFICATION**

(45) Date of publication and mention of the grant of the patent:
**26.09.2007 Bulletin 2007/39**

(21) Application number: **04745141.4**

(22) Date of filing: **19.07.2004**

(51) Int Cl.:
*A61K 31/4045* (2006.01) *A61K 31/00* (2006.01)
*A61P 9/10* (2006.01) *A61P 39/06* (2006.01)
*A61K 31/704* (2006.01)

(86) International application number:
**PCT/IN2004/000216**

(87) International publication number:
**WO 2005/009419 (03.02.2005 Gazette 2005/05)**

(54) **USE OF 5-METHOXYTRYPTAMINE AS A CARDIOPROTECTIVE AGENT**
VERWENDUNG VON 5-METHOXYTRYPTAMIN ALS KARDIOPROTEKTIVES MITTEL
UTILISATION DE LA 5-METHOXYTRYPTAMINE COMME AGENT CARDIOPROTECTEUR

(84) Designated Contracting States:
**AT BE BG CH CY CZ DE DK EE ES FI FR GB GR HU IE IT LI LU MC NL PL PT RO SE SI SK TR**

(30) Priority: **25.07.2003 US 627398**

(43) Date of publication of application:
**10.05.2006 Bulletin 2006/19**

(73) Proprietor: **Dabur Research Foundation**
**Ghaziabad, 201 010,**
**Uttar Pradesh (IN)**

(72) Inventors:
- **MUKHERJEE, Rama, Dabur Research Foundation Ghazibad 201 010 (Uttar Pradesh) (IN)**
- **SINGH, Anu T., Dabur Research Foundation Ghazibad 201 010 (Uttar Pradesh) (IN)**
- **DUTTA, Kakali, Dabur Research Foundation Ghazibad 201 010 (Uttar Pradesh) (IN)**
- **MAIKAP, G. C., Dabur Research Foundation Ghazibad 201 010 (Uttar Pradesh) (IN)**
- **SHUKLA, Anil Kumar, Dabur Research Foundation Ghazibad 201 010 (Uttar Pradesh) (IN)**
- **KHATTAR, Dhiraj, Dabur Research Foundation Ghazibad 201 010 (Uttar Pradesh) (IN)**
- **BURMAN, Anand C., Dabur Research Foundation Ghazibad 201 010 (Uttar Pradesh) (IN)**

(74) Representative: **Bailey, Lindsey et al**
**Frank B. Dehn & Co.**
**St Bride's House**
**10 Salisbury Square**
**London EC4Y 8JD (GB)**

(56) References cited:
**WO-A-01/49286**
**WO-A-02/22573**
**WO-A-96/27658**
**WO-A-20/04045509**
**US-A- 5 985 293**
**US-A1- 2002 173 511**
**US-A1- 2003 105 318**

- **DATABASE CA [Online] CHEMICAL ABSTRACTS SERVICE, COLUMBUS, OHIO, US; VINOGRADOV, V. M. ET AL: "Effect of antioxidants on the resistance to hypoxia, and some aspects of energy metabolism" XP002311445 retrieved from STN Database accession no. 1970:53551 & MATER. SIMP. "VYSOKOGOR'E KRASNAYA KROV" KONF. "VYSOKOGOR'E LEK." , 173-4. EDITOR(S): ALIEV, M. A. PUBLISHER: IZD. "ILIM", FRUNZE, USSR. CODEN: 21XFAX, 1968,**
- **DATABASE CA [Online] CHEMICAL ABSTRACTS SERVICE, COLUMBUS, OHIO, US; ROGOVA, L. S. ET AL: "Antiarrhythmic properties of some indole alkylamines" XP002271167 retrieved from STN Database accession no. 70:2017 & BYULLETEN EKSPERIMENTAL'NOI BIOLOGII I MEDITSINY (1968), 66(10), 58-60, 1968,**
- **SAXENA P R ET AL: "5-Hydroxytryptamine: A chameleon in the heart" TRENDS IN PHARMACOLOGICAL SCIENCES 1991 UNITED KINGDOM, vol. 12, no. 6, 1991, pages 223-227, XP001176658 ISSN: 0165-6147**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- **DATABASE WPI Week 197909 12 May 1978 (1978-05-12), Derwent Publications Ltd., London, GB; AN 1979-17341B XP002271168 KRASNYKU ET AL.: "5-Methoxytryptamine pharmaceutical - used as radiation sickness protective agent" & SU 227 537 A (ZHEREBCHENKO P.G.) 12 May 1978 (1978-05-12)**
- **REITER RUSSEL J ET AL: "Melatonin: Reducing the toxicity and increasing the efficacy of drugs." JOURNAL OF PHARMACY AND PHARMACOLOGY, vol. 54, no. 10, October 2002 (2002-10), pages 1299-1321, XP008027671 ISSN: 0022-3573**
- **ABD EL-AZIZ M A ET AL: "Potential protective role of angiotensin-converting enzyme inhibitors captopril and enalapril against adriamycin-induced acute cardiac and hepatic toxicity in rats" JOURNAL OF APPLIED TOXICOLOGY, vol. 21, no. 6, November 2001 (2001-11), pages 469-473, XP008040546 ISSN: 0260-437X cited in the application**
- **HARA M ET AL: "Administration of melatonin and related indoles prevents exercise-induced cellular oxidative changes in rats." BIOLOGICAL SIGNALS, (1997 MAR-APR) 6 (2) 90-100., March 1997 (1997-03), XP008027668**
- **NG T.B. ET AL: "Antioxidative and free radical scavenging activities of pineal indoles." JOURNAL OF NEURAL TRANSMISSION, (2000) 107/11 (1243-1251)., 2000, XP001176659 cited in the application**


Remarks:

The file contains technical information submitted after the application was filed and not included in this specification

## Description

## FIELD OF INVENTION

The invention relates to the use of 5-methoxy tryptamine or a salt thereof for the manufacture of a medicament for the prevention and/or treatment of Doxorubicin induced cardiotoxicity or cardiac tissue injury\ischemia\stress\hypoxia. Serum creatine kinase-MB levels of a patient are reduced by administering to the patient an effective amount of 5-methoxytryptamine or a salt thereof.

## BACKGROUND OF INVENTION

The pineal gland secretes a number of pineal indoles including melatonin, methoxytryptophol, methoxytryptamine and other methoxyindoles and hydroxyindoles. The most extensively studied of the pineal indoles is melatonin ( Burkhard, Poeggeler et al. J. Pineal. Res. 2002, 33 : 20- 30). The other pineal indoles have not been examined to the same depth. 5-Methoxytryptamine, one of the pineal indoles, is an agonist of 5- Hydroxy Tryptamine viz. Serotonin. It binds to the 5 - $HT_6$ receptor subtype of serotonin, which may be exclusively localized to the central nervous system.

5-Methoxytryptamine is widely known to be an effective radioprotective agent (Kuna, P. et.al.,Radiobiologia, Radiotherapia 1983, 24 (3), 365 -76; Rozhdestvenskii, L. M. and Grozdov S.P. Radiobiologiya, 1979, 19(6), 868 - 75; Parzyck, DC. et al. Radiochemical and Padioanalytical Letters, 1974, 17( 5-6), 351- 358; Streffer, C. and Fluegel, M. Strahlentherapie , 1973, 146 (4), 444- 449 and Feher, Imre et al., Int. J. Radiat. Biol, 1968, 14 (3), 257 - 262.)

5-Methoxytryptamine is also known to exert significant immunomodulating effects on cytokine secretion, consisting of inhibition of tumour necrosis factor alpha secretion with an anti - cachectic property ( Sacco, S. et al. Eur. J. Pharmacol. 1998, 34 : 249 - 255) and stimulation of IL2 and gamma interferon release with the following antitumour immunomodulatory effects ( Sze, S.F. et al. J. Neural. Transm. Gen. Sect. 1993, 94, 115-126).

5- Methoxytryptamine has also been shown to possess free radical scavenging and anti-oxidative effects in hepatic and kidney tissues homogenates, mediated by a reduction in lipid peroxidation ( Ng, T.B et. al , J. Neural Transmission 2000, 107(11), and this may be on account of its 5- methoxylic group ( Chan, T.Y. and Tang, P.L., J. Pineal. Res. 1993, 14 : 27- 33).

Recently, pineal indoles like 5-Methoxytryptamine have been reported to possess oncostatic activity (Paolo Lissoni etal, Neuroendocrinol Letts, 2000 : 21 : 319 - 323).

Anthracycline antineoplastics are amongst the most active anticancer drugs and are effective against malignancies like leukemias, lymphomas and many solid cancers. These include Doxorubicin ( sold under the trademark ADRIAMYCIN, NSC 123127, From Adria Laboratories, Columbus, Ohio), Daunorubicin, Epirubicin, THP-Adriamycin and Idarubicin. Doxorubicin is the drug of choice, alone or in combination with other chemotherapeutic agents, in the treatment of metastatic adenocarcinoma of the breast, carcinoma of the bladder, bronchogenic carcinoma, neuroblastoma, and metastatic thyroid carcinoma. It exerts its antitumour effects due to inhibition of DNA replication by intercalating between base pairs and/or steric inhibition of RNA activity.

Cardiac tissue injury is the major limitation in the use of doxorubicin ( Weiss, R.B., Semin. Oncol. 19, 670 - 686, 1992). The risk of developing cardiomyopathy becomes unacceptably high beyond the cumulative dose of 550 mg/m2 (Lefrak et al., Cancer 1973, 32, 302- 314). In addition to clinical heart failure, cardiotoxicity encompasses clinical cardiotoxicity such as cardiac tissue injury\ischemia\stress\ hypoxia\congestive heart failure and\or cardiac arrhythmias, and subclinical cardiotoxicity such as that detected by pathologic changes in cardiac biopsy or decrease in ventricular ejection fractions.

Thus, it has been found that doxorubicin treatment often must be terminated before the maximum effective cumulative dose has been administered to a patient bearing a neoplasm, because of the development of life-threatening cardiomyopathy. Thus, while doxorubicin is considered a highly effective anti-tumor agent, this effectiveness is significantly reduced by the concomitant cardiotoxicity encountered with use of the drug.

Doxorubicin induced cardiotoxicity is mediated through several different mechanisms including lipid peroxidation ( Bordoni, A. et al., Biochim. Biophys. Acta 1999, 1440: 100- 106), free radical formation ( Yin, X. et.al., Biochem. Pharmacol. , 1998, 56: 87- 93, Hershko, C. et al, Leuk. Lymphoma 1993, 11 : 207 -214 ), mitochondrial damage (Cini Neri, G. et al., Oncology 1991, 48: 327- 333), and iron dependent oxidative damage to biological macromolecules (Thomas, C.E. and Aust, S.D., Arch. Biochem. Biophys. 1986, 248: 684-689).

The complete mechanisms for doxorubicin and other anthracycline-induced cardiotoxicity are not completely understood. Three intracellular mechanisms are ascribed to Anthracyclines : interactions with DNA synthesis, binding to cell membranes and altering membrane functions, and intracellular $Na^+$ & $Ca^{2+}$ concentrations and stimulation of lipid peroxidation to form oxygen radicals (Young, R.C et. al, N. Engl. J. Med. 1981 , 305: 139-153). It also may induce apoptosis in cardiomyocytes ( Arola, O.J. et al., Cancer Res., 2000 Apr 1, 60 (7) : 1789- 1792). Further on, Doxorubicin induced cardiac tissue injury produces significant elevation in serum levels of Creatine Kinase MB isoform ( M A Abd El

- Aziz etal, Journal of applied Toxicology 21, 469 - 473 , 2001 , (Xiaoping Luo etal, Biochimica et Biophysica Acta 1360 (1997) 45 - 52)

The effects of Doxorubicin on intracellular calcium homeostasis seems to be especially associated with the development of chronic cardiomyopathy (Young, RC. et al., N. Engl. J. Med. 1981, 305: 139-153). Further Doxorubicin inhibits $Na^+/Ca^{2+}$ exchanger (Caroni, P., et al., FEBS Lett 1981, 130 : 184 - 186), the oxygen consumption and the ATP production of mitochondria in *in vitro* rat heart preparation (Bachmann, E., et al., Agents Action 1975, 5: 383- 393). Chronic dilated cardiomyopathy which can be induced by long term Doxorubicin treatment causes an upregulation of α and β adrenergic system as well as of the renin - angiotensin system ( Kanda, T. et al., Eur. Heart J. 1994 , 15, 686-690 and Morgan, H.E., Circulation, 1993, 87 IV4 - IV6). Existing literature supports the view that one of the mechanisms may involve drug induced, cytotoxic, free radical formation (Buja et al. , Cancer, 1973, 32, 771-778; Arena, E., et al. , Int. Res. Commun. Syst. Med. Sci., 1974, 2, 1053-1061; Bristow, M.R. et al. Cardiovasc. Pharmacol., 1980, 2, 487-515). Further Doxorubicin administration is associated with a decrease in the presence of the endogenous antioxidants. Doxorubicin directly depresses cardiac glutathione peroxidase activity, the major defense against free-radical damage.

Pharmacological development of novel cardioprotectives has involved the exploration of diverse classes of molecules.

At present, Dexrazoxane (ICRF -187, Zinecard), is an iron chelator, and is the only drug in human clinical use to reduce Doxorubicin induced cardiotoxicity (Swain, S.M. et al., J. Clin. Oncol., 1997 :15 :1333 -1340, and Swain, S.M. et. al., J. Clin. Oncol., 1997,15:1318 -1332).

Reiter Russel J et al.: "Melatonin: Reducing the toxicity and increasing the efficacy of drugs". Journal of Pharmacy and Pharmacology, vol. 54, No. 10, October 2002 (2002-10), pages 1299-1321, suggests that melatonin can reduce the toxicity associated with the administration of a large number of antineoplastic agents including anthracycline glycosides.

Diverse classes of molecules or active principles of plants have shown cardioprotective activities for Doxorubicin induced cardiotoxicity in animal models. These include lipid lowering drugs like Lovastatin ( Feleszko, W. et al., Clin. Cancer. Res. Vol 6,2044 - 2052, May 2000 ) probucol ( Li, T; and Singal, P., Circulation, 2000,102:2105 -2110), cytoprotective drugs like Amifostine ( Jahnukainen, K. et al., Cancer Research, 61,6423 - 6427, September 1,2001), free radical scavengers like Vitamin E or N-acetylcysteine, calcium channel antagonists like Amlodipine (Yamanaka,S. et al., J. Am. Coll. Cardiol. 2003, Mar. 5,41(5): 570-878 ),non selective β adrenoceptor blocker and vasodilator like Carvedilol ( Santos, D.L. et al, Toxicol. Appl. Pharmacol, 2002 Dec 15,185(3): 218 -227), Angiotensin converting Enzyme inhibitors like Captopril and Enalapril ( El Aziz, M. A. et al., J. Appl. Toxicol, 21,469 - 473,2001) and plant extracts like curcumin (Venkatesan, N., Br. J. Pharmacol. 1998, Jun, 124 (3); 425 -427).

There are several enzyme markers as well as serum markers indicative of tissue injury\ischemia\stress\hypoxia. These include enymes like Creatine Kinase & its myocardium specific isoform like Creatine Kinase MB ( CK- MB), Lactate Dehydrogenase ( LDH) ,Superoxide Dismutase (SOD) & membrane lipid peroxidation.

Superoxide Dismutase is the most important enzyme involved in the primary cellular defense. It decomposes the superoxide radicals to hydrogen peroxide which is in turn consumed by multiple enzymes such as catalase and glutathione peroxidase (Halliwell, B., Lancet 1994,344: 721- 724). Superoxide Dismutase is induced by hyperoxia (Crapo, J.D. and Tierney, D.F., Am. J. Physiol. 1974,226:1401- 1407), irradiation (Oberley, L.W. et al., Arch. Biochem. Biophys., 1987,:54, 69- 80) and changes in cellular redox status (Warner, B.B. et al., Am. J. Physiol., 1996:271, L 150- L158). Experiments have been conducted using Superoxide Dismutase therapy for the treatment of myocardial ischemia (Downey, J.M. et al., Free Radic. Res. Commun. 1991, 12- 13 Pt2: 703- 720).

Creatine kinase is an enzyme, which is readily measured in the blood of any individual with muscular tissue trauma or disease (Robinson, David J. et al., J. of Emergency Medicine , Vol 17, No. 1, pp 95- 104, 1999) . Further the cardiac specific isozyme of Creatine Kinase , CK- MB is produced exclusively in the myocardium, with very small amounts measured in the small intestine, tongue, diaphragm, uterus and prostate (Tsung, S. , Clin. Chem. 1976, 22, 173). Creatine Kinase activity is well accepted index of cardiac injury ( Xiaoping Luo etal, Biochimica et Biophysica Acta 1360 ( 1997) 45 -52) .CK- MB measurements thus provide for a specific marker for identifying cardiac tissue damage and has become the " gold standard" for assessing myocardial infarction (Gillum, R.F. et al., Am Heart J , 1984, 108 : 150- 158). It is the only serum marker currently accepted in the World Health Organization (WHO) guidelines for the diagnosis of acute myocardial infarction (Gillum, R.F., et al., Am. Heart. J., 1984, 108 : 150-158).

Doxorubicin treatment produces significant elevation in CK - MB levels in cardiac toxicity responses (M A Abd El - Aziz etal, Journal of applied Toxicology 21, 469 - 473 , 2001 ), (Xiaoping Luo etal, Biochimica et Biophysica Acta 1360 (1997) 45 -52 ,) which may be reduced by cardioprotective iron chelating molecules like Deferoxamine ( Sherif Y. Saad, Pharmaceutical Research Vol 43 , no. 3, 2001, 211 - 217 ). Further these protective effects related to decrease in CK MB levels are reflected in defined histopathological protection in the cardiac tissue (Sherif Y. Saad, Pharmaceutical Research Vol 43 , no. 3, 2001, 211 - 217). Similar cardioprotection has been reported by the chronic pretreatment with Dexrazoxane , also called ICRF-187( Tai K. Yeung etal, Cancer Chemother Pharmacol 1992 , 30 : 58 - 64)

The enzyme Lactate dehydrogenase (LDH) catalyzes the reversible transfer of two electrons and hydrogen

ion from lactate to NAD resulting in pyruvate and NADH. LDH is distributed in heart, kidney, brain, stomach and skeletal muscle. After an acute myocardial infarction (AMI), serum LDH activity starts to rise 12- 18 hours after the onset of symptoms, and returns to normal by 6 - 10 days (Wolf, P.L., Clin. Lab. Med. 1989, 9 : 655). Elevated LDH levels are associated with a variety of pathological conditions.

5-Methoxy tryptamine (5-MT, Structure-I), and its salts, the subject of this invention, show promise as cardioprotectors for Doxorubicin induced cardiac tissue injury/ischemia/stress/hypoxia in animal studies.

**SUMMARY OF THE INVENTION**

The present invention is directed to the use of 5-methoxy tryptamine or a salt thereof for the manufacture of a medicament for the prevention and/or treatment of doxorubicin induced cardiotoxicity in a subject.

**DETAILED DESCRIPTION OF THE INVENTION**

5- Methoxytryptamine (available from M/s Aldrich) is represented by Structure I

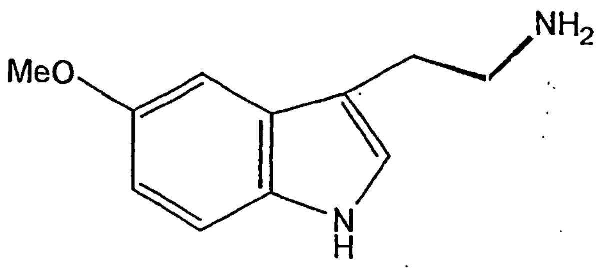

**Structure -I**

The present invention is concerned with the presentation of 5-Methoxytryptamine and its salts in pharmaceutically acceptable form to patients undergoing doxorubicin treatment with a view to treat or prevent injury/ischemia/stress/hypoxia to myocardial tissue. In the compositions used according to this invention 5-Methoxytryptamine remains physically and chemically stable and can be administered in various dosage forms at the drug dose meant to be effective to exhibit clinically significant cardioprotective activity.

5-Methoxytryptamine can be used to prevent and/or treat Doxorubicin induced cardiac toxicity, myocardial ischemia, myocardial infarction or heart failure.

The prevention and/or treatment comprises or consists of administering orally, parenterally, or systemically to the mammal a therapeutically effective dose of 5-Methoxy tryptamine or its salts. An effective dose of 5-methoxy tryptamine or its salts thereof ranges from 0.7 to 7.0 mg/kg body weight, more preferably 1.2 - 5.0 mg/kg body weight, with the dose being dependent on the extent of effects sought and the manner of administration. This invention includes the use of pharmaceutical compositions, containing 5-Methoxytryptamine or its pharmaceutically acceptable salts alongwith or in combination with one or more carriers, diluents, excipients and/or additives. The composition typically contains an amount of 5-Methoxytryptamine or a salt thereof effective to achieve the intended purpose. The unit dosage of a composition typically ranges from 5 mg - 500mg of 5-Methoxytryptamine or a salt thereof. An effective amount means that amount of a drug or pharmaceutical agent that will elicit the biological or medical response of a tissue, system, animal or human that is sought. In accordance with good clinical practice, it is preferred to administer the composition at a dose that will produce the effects sought without causing undue harmful side effects.

The term "salts" refers to salts prepared from pharmaceutically non-toxic bases including organic bases and inorganic bases. Representative salts include the following: acetate, ascorbate, benzoate, citrate, oxalate, stearate, trifluoroacetate, succinate, tartarate, lactate, fumarate, gluconate, glutamate, phosphate/diphosphate, and valerate. Other salts include Ca, Li, Mg, Na, and K salts, halides, salts of amino acids such as lysine or arginine; guanidine,

ammonium, substituted ammonium salts or aluminium salts.

The salts of 5-Methoxy tryptamine may be prepared by methods known to those skilled in the art.

5-Methoxytryptamine or its salts can be administered orally to human cancer patients by incorporating in a flavoured/sweetened syrup base.

5-Methoxytryptamine or its salts can be dissolved in a small amount of suitable solvent like water or alcohol. 5-Methoxytryptamine can be adsorbed onto inert excipients like colloidal silica to convert into a solid form that can be dispensed in a sachet, ampoule, vial, filled into hard gelatin capsules or into soft gelatin capsules. 5-Methoxytryptamine can be compressed into tablets with or without the addition of excipients. The formulations can be in the form of tablets, powders, capsules, lozenges, solutions, syrups, aqueous or oily suspensions, elixirs, implants, or aqueous or nonaqueous injections or any other forms that are pharmaceutically acceptable.

All the above delivery systems may contain added auxiliary agents such as fillers, diluents, preservatives or stabilizers.

The composition may be administered either alone or as a mixture with other therapeutic agents.

The *in vitro* and *in vivo* activity of the 5-Methoxytryptamine and its salts may be determined by standard assays that determine their free radical scavenging properties, effects on lipid peroxidation in cardiac homogenates, effects of the compounds on antioxidant enzymes viz. Superoxide Dismutase, Catalase, and on antioxidant peptides as on reduced Glutathione.

We have investigated the effect of 5-Methoxytryptamine on scavenging of free radicals *in vitro,* effect of 5-Methoxytryptamine on lipid peroxidation in live myocardial tissue, effect of 5- Methoxytryptamine on Superoxide Dismutase enzyme activity in live myocardial tissue, effect of 5-Methoxytryptamine on lipid peroxidation in live hepatic tissue, effect of 5-Methoxytryptamine on anticancer activity of Doxorubicin *in vitro;* effect of 5-Methoxytryptamine on circulating levels of Creatine Kinase-MB (CK-MB) in Doxorubicin treated animals, and effect of 5-Methoxy tryptamine on circulating levels of Lactate Dehydrogenase (LDH) enzyme in Doxorubicin treated animals.

The present invention will now be illustrated by the following examples. All examples given below as illustrated were carried out using Doxorubicin (referred as Dox) and 5 Methoxytryptamine referred as 5MT.

**Example 1:**

**Effect of 5 Methoxytryptamine on scavenging of free radicals *in vitro.***

The free radical scavenging potential of 5- Methoxytryptamine was evaluated by the 1,1 diphenyl - 2 picryl hydrazyl (DPPH) assay as described (Hycon, Lee et al, Arch. Pharm. Res. 19 (3), 223 - 227). Briefly 0.2 mM solution of 1,1 diphenyl - 2 picryl hydrazyl was prepared in 100% methanol and immediately protected from light and kept at - 20° C. 5- Methoxytryptamine was dissolved in 3.5 % ethanol in normal saline and screened for its radical scavenging activity in concentration ranging from 1-1000 ug/ml. 100ul of 0.2 mM DPPH was incubated with 100ul of varying concentrations of 5-Methoxy tryptamine in 96 well tissue culture plates for 20 seconds at room temperature. All experiments were carried out in triplicates. 3.5% ethanol in normal saline was similarly incubated with 0.2 mM DPPH in control experiments for evaluating the effect of the vehicle on radical scavenging. The change in absorbance of DPPH incubated with varying concentrations of 5- Methoxy tryptamine of the vehicle was read at 517nM for every 60 secs for 5 minutes. The absorbance of 0.2mM DPPH taken after incubating for 5 minutes at room temperature was the blank O.D. The percent free radical scavenging ability of 5- Methoxy tryptamine was calculated as defined below.

$$\text{Percent Free Radical scavenging} = \frac{\text{Blank OD at 5 minutes} - \text{Sample OD at 5 minutes}}{\text{Blank OD at 5 minutes}} \times 100$$

Table 1 shows the percent radical scavenging ability of 5- Methoxytryptamine *in vitro.* As shown in Table 1, 5-Methoxytryptamine scavenges free radicals in a concentration ranging from 31 - 1000ug/ml. It scavenges a maximal of 88.25 % of free radicals at a concentration of 1000ug /ml *in vitro.*

**Table 1 : Mean percent radical scavenging by 5-Methoxytryptamine *in vitro.***

| S.no | Concentration of 5 Methoxytryptamine | Mean percent Radical scavenging |
|---|---|---|
| 1 | 31 ug/ml | 10 ± 4.3 |
| 2 | 62.5 ug/ml | 25 ± 5.0 |

(continued)

| S.no | Concentration of 5 Methoxytryptamine | Mean percent Radical scavenging |
|---|---|---|
| 3 | 125ug/ml | 75.4 ± 5.5 |
| 4 | 250 ug/ml | 85.24 ± 4.8 |
| 5 | 500ug/ml | 87.4 ± 5.0 |
| 6 | 1000ug/ml | 88.25 ± 5.1 |

**Example 2:**

**Effect of 5 Methoxytryptamine on lipid peroxidation in live myocardial tissue.**

The effect of 5- Methoxytryptamine on lipid peroxidation in Doxorubicin treated myocardial tissue was quantitated by Thiobarbituric acid reactive substances based assay as described (Uchiyama and Mihara, M., Anal. Biochem. 86, 271 - 278, 1978). Briefly male Wistar rats of the age group 5-6 weeks were maintained on normal rat pellets *ad libitum.* Rats were divided into four groups viz Groups I ,II, III and IV.

Group I : Untreated
Group II: Animals treated with Doxorubicin
Group III: Animals treated with 5- Methoxytryptamine and Doxorubicin
Group IV: Animals treated with 5 -Methoxytryptamine

Each group consisted of 5 animals. 30 mg/ kg body weight of Doxorubicin was administered intraperitoneally to animals in Groups II and III. The animals comprising group III, were injected intraperitoneally with 5- Methoxytryptamine in concentration ranging from 8.5 - 35 mg/kg body weight 30 minutes prior to the Doxorubicin treatment. The animals comprising group IV, were injected intraperitoneally with 5-Methoxytryptamine in concentration ranging from 8.5 - 35 mg/kg body weight. 24 hours later, the beating hearts of the animals were excised by decapitation. The heart tissue was washed in ice cold saline twice, weighed and snap frozen at -70°C for assaying for Lipid peroxidation.

Briefly 200 mg of the live rat myocardial tissue was excised and homogenized in 2 ml of ice cold 10% Trichloro acetic acid buffer (TCA) buffer. To 200 μl of the homogenate thus obtained, 200ul of 8.1 % SDS , 1.5 ml of 20% Acetic acid, 1.5ml of 0.8% of Thiobarbituric acid (TBA) and 1.0 ml of water was added in glass test tubes. The tubes were heated at 95°C for 60 minutes. The mixture was cooled and diluted with 1 ml of double distilled water. A mixture of n - butanol and pyridine was prepared fresh in the ratio of 15: 1 respectively. 5 ml of the n - butanol- pyridine mix was added to each tube containing the cardiac tissue homogenates. The tubes were centrifuged at 3000 rpm for 10 minutes at 4°C. 200ul of the coloured liquid was collected from at the interphase of the aqueous and organic layers and the absorbance was measured spectrophotometrically at 532 nm. The control experiments contained only the TCA buffer treated similarly. The standard tubes contained Malonaldehyde in concentrations ranging from 2.5 -25uM. Malonaldehyde was dissolved in double distilled water. All experiments were carried out in triplicates. The extent of lipid peroxidation in the cardiac homogenates was expressed as uM / gm of the cardiac tissue. The extent of lipid peroxidation was calculated for the cardiac tissues of the animals comprising Groups I, II, III and IV. As shown in Table 2, 5-Methoxytryptamine in concentrations ranging from 8.5 - 35 mg/ kg inhibits the lipid peroxidation in *vivo* in rat myocardial tissue treated with 30mg/kg of Doxorubicin. Further treatment with 5 Methoxytryptamine alone in concentrations ranging from 8.5 - 35mg/kg did not alter the lipid peroxidation in *vivo*

**Table 2 : Lipid peroxidation (umoles/gm) in Doxorubicin treated rat myocardium *in vivo***

| Group | Treatment | Dose | | LP ( umoles/g) |
|---|---|---|---|---|
| | | Doxorubicin | 5 MT | |
| I | Untreated | Nil | Nil | 553 ± 10.5 |
| II | Dox | 30mg/kg | Nil | 783 ± 21.0 |
| III | A (Dox & 5MT) | 30 mg/kg | 8.5 mg/kg | 613 ± 20.1 |
| | B (Dox & 5MT) | 30 mg/kg | 17mg/kg | 625 ± 10.5 |
| | C (Dox & 5MT) | 30 mg/kg | 35 mg/kg | 650 ± 11.2 |

(continued)

| Group | Treatment | Dose | | LP ( umoles/g) |
|---|---|---|---|---|
| | | Doxorubicin | 5 MT | |
| IV | A (5MT) | Nil | 8.5 mg/kg | 535 ± 12.5 |
| | B (5MT) | Nil | 17mg/kg | 500 ± 14.5 |
| | C (5MT) | Nil | 3 5 mg/kg | 513 ± 12.3 |

**Example 3:**

**Effect of 5-Methoxytryptamine on Superoxide Dismutase enzyme activity in live myocardial tissue.**

The effect of 5-Methoxy tryptamine on Superoxide Dismutase activity in myocardial tissue was calculated as described ( Kahhar et al., Indian Journal of Biochem. and Biophys. Vol. 21, Apr. 1984, 130 - 132 ). Briefly male Wistar rats of the age group 5 - 6 weeks were maintained on normal rat pellets *ad libitum.* Rats were divided into four groups viz. Groups I , II, III and IV.

Group I : Untreated
Group II : Animals treated with Doxorubicin
Group III: Animals treated with 5- Methoxytryptamine and Doxorubicin
Group IV : Animals treated with 5- Methoxytryptamine

Each group consisted of 5 animals. 30 mg/ kg body weight of Doxorubicin was administered intraperitoneally to animals in Groups II and III. The animals comprising group III, were injected intraperitoneally with 5-Methoxytryptamine in concentration ranging from 8.5 - 35 mg/kg body weight 30 minutes prior to the Doxorubicin treatment. The animals comprising group IV, were injected intraperitoneally with 5 Methoxytryptamine in concentration ranging from 8.5 - 35 mg/kg body weight. 24 hours later, the beating hearts of the Animals were excised by decapitation. The heart tissue was washed in ice cold saline twice, weighed and frozen for assaying Superoxide Dismutase activity.

Briefly, 200 mg of rat myocardial tissue was excised and homogenized in 2ml of ice cold Tris sucrose buffer (pH 7.4). The homogenate was centrifuged at 10000 rpm, at 4°C for 10 minutes, and the supernatent carefully aspirated and collected. For each experiment, 1.2 ml of sodium pyrophosphate buffer was taken in clean glass tubes. To this 100ul of 186 uM Phenazine methosulphate solution, 300ul of a 300uM solution of Nitroblue tetrazolium and 600ul of double distilled water was added and mixed well. 600ul of the supernatent obtained earlier was added per tube. A solution of NADH of the concentration 780uM was freshly prepared for the experiments. The reaction in the tubes was initiated by the addition of 200ul of a 780μM solution of NADH per tube. The tubes were incubated for 90 seconds at room temperature. The reaction was stopped by the addition of 1ml of 100% glacial acetic acid per tube and the absorbance measured spectrophotometrically at 560nM. All experiments were carried out in triplicates. The control experiments contained only ice cold Tris Sucrose buffer (pH 7.4) treated identically. The standard tubes contained the enzyme Superoxide Dimutase dissolved in double distilled water in concentrations ranging from 0.1U to 20Units treated identically as above. The enzyme activity in the tissue homogenates was quantitated by rate of decrease in optical density at 560nm, and expressed as units/ mg protein. The Superoxide Dismutase activity was calculated for the cardiac tissues of the animals comprising Groups I, II, III and IV. As shown in Table 3, treatment with 5 - Methoxytryptamine in concentrations ranging from 8.5-35 mg/ kg increases the Superoxide Dismutase activity *in vivo* in rat myocardium treated with 30mg/kg of Doxorubicin. Further treatment with 5- Methoxytryptamine alone in concentrations ranging from 8.5 - 35mg/kg did not alter the Superoxide Dismutase activity in *vivo.*

**Table 3**

| Superoxide Dismutase ( U/mg) in Doxorubicin treated rat myocardium *in* vivo | | | | |
|---|---|---|---|---|
| **Group** | **Treatment** | **Dose** | | **SOD ( U/mg)** |
| | | Doxorubicin | 5 MT | |
| I | Untreated | Nil | Nil | 35.1 ± 2.5 |
| II | Dox | 30mg/kg | Nil | 3.3 ± 0.2 |

(continued)

| Superoxide Dismutase ( U/mg) in Doxorubicin treated rat myocardium *in* vivo | | | | |
|---|---|---|---|---|
| **Group** | **Treatment** | **Dose** | | **SOD ( U/mg)** |
| | | Doxorubicin | 5 MT | |
| III | A (Dox & 5MT) | 30 mg/kg | 8.5 mg/kg | 5.95 ± 1.3 |
| | B ( Dox & 5MT) | 30 mg/kg | 17mg/kg | 24.3 ± 2.5 |
| | C (Dox& 5MT) | 30 mg/kg | 35 mg/kg | 28.1 ± 3.0 |
| IV | A (5MT) | Nil | 8.5 mg/kg | 32 ± 2.5 |
| | B (5MT) | Nil | 17mg/kg | 30 ± 2.9 |
| | C (5MT) | Nil | 35 mg/kg | 34 ± 4.5 |

**Example 4 (Reference)**

**Effect of 5 Methoxytryptamine on lipid peroxidation in live hepatic tissue.**

The effect of 5 Methoxytryptamine on lipid peroxidation in Doxorubicin treated hepatic tissue was quantitated by Thiobarbituric acid reactive substances based assay as described (Uchiyama and Mihara, M., Anal Biochem. 86, 271 - 278, 1978). Briefly male Wistar rats of the age group 5 - 6 weeks were maintained on normal rat pellets *ad libitum.* Rats were divided into four groups viz. Groups I , II , III and IV.

Group I : Untreated
Group II : Animals treated with Doxorubicin
Group III: Animals treated with 5- Methoxytryptamine and Doxorubicin.
Group IV : Animals treated with 5- Methoxytryptamine

Each group consisted of 5 animals. 30 mg/ kg body weight of Doxorubicinwas administered intraperitoneally to animals in Groups II and III. The animals comprising group III, were injected intraperitoneally with 5- Methoxytryptamine in concentrations ranging from 8.5 - 35 mg/kg body weight 30 minutes prior to the Doxorubicin treatment. The animals comprising group IV, were injected intraperitoneally with 5-Methoxytryptamine in concentrations ranging from 8.5 - 35 mg/kg body weight. 24 hours later, the liver tissue of the animals were excised. The liver tissue was washed in ice cold saline twice, weighed and snap frozen at -70°C for assaying for Lipid peroxidation.

Briefly 200 mg of the rat liver tissue was excised and homogenized in 2 ml of ice cold 10% Trichloro acetic acid buffer (TCA) buffer. To 200 ul of the homogenate thus obtained, 200ul of 8.1 % SDS , 1.5 ml of 20% Acetic acid, 1.5ml of 0.8% of Thiobarbituric acid (TBA) and 1.0 ml of water was added in glass test tubes. The tubes were heated at 95°C for 60 minutes. The mixture was cooled and diluted with 1 ml of double distilled water. A mixture of n - butanol and pyridine was prepared fresh in the ratio of 15: 1 respectively. 5 ml of the n - butanol- pyridine mix was added to each tube containing the liver tissue homogenates. The tubes were centrifuged at 3000 rpm for 10 minutes at 4°C. 200ul of the colored liquid was collected from the interphase of the aqueous and organic layers and the absorbance was measured spectrophotometrically at 532 nm. The control experiments contained only the TCA buffer treated similarly. The standard tubes contained Malonaldehyde in concentrations ranging from 2.5 -25uM. Malonaldehyde was dissolved in double distilled water. All experiments were carried out in triplicates. The extent of lipid peroxidation in the liver homogenates was expressed as uM / gm of the liver tissue. The extent of lipid peroxidation was calculated for the liver tissues of the animals comprising Groups I, II, III and IV. As shown in Table 4, 5- Methoxytryptamine in concentrations ranging from 8.5 - 35 mg/ kg inhibits the lipid peroxidation *in vivo* in rat liver tissues treated with 30mg/kg Doxorubicin. Further treatment with 5 Methoxy tryptamine alone in concentrations ranging from 8.5 - 35 mg/kg does not alter the lipid peroxidation in *vivo.* Thus 5- Methoxytryptamine reduces the Doxorubicin induced peroxidative damage to the liver tissue.

Table 4

| Lipid peroxidation (umoles/gm) in Doxorubicin treated rat liver tissue *in vivo* | | | | |
|---|---|---|---|---|
| **Group** | **Treatment** | **Dose** | | **LP (umoles/g)** |
| | | Doxorubicin | 5 MT | |

(continued)

| Lipid peroxidation (umoles/gm) in Doxorubicin treated rat liver tissue *in vivo* | | | | |
|---|---|---|---|---|
| **Group** | **Treatment** | **Dose** | | **LP (umoles/g)** |
| I | Untreated | Nil | Nil | 627 ± 11.5 |
| II | Dox | 30mg/kg | Nil | 799 ± 11.0 |
| III | A (Dox & 5MT) | 30 mg/kg | 8.5 mg/kg | 613 ± 210.1 |
| | B ( Dox & 5MT) | 30 mg/kg | 17mg/kg | 625 ± 11.5 |
| | C (Dox & 5MT) | 30 mg/kg | 35 mg/kg | 650 ± 15.2 |
| IV | A (5MT) | Nil | 8.5 mg/kg | 601 ± 16.5 |
| | B (5MT) | Nil | 17mg/kg | 625 ± 15.5 |
| | C (5MT) | Nil . | 35 mg/kg | 630 ± 16.3 |

In view of the reported role of free radicals in causing tissue damage to diverse tissues as described earlier, the use of 5- Methoxytryptamine for the said purpose may be extended for the treatment for similar damage to other tissues viz, the brain, intestine , kidney, lung, and pancreas.

**Example 5**

**Effect of 5 -Methoxytryptamine on circulating levels of Creatine Kinase - MB (CK- MB) in a subchronic study in Doxorubicin treated animls.**

The effect of 5- Methoxy tryptamine on circulating CK-MB levels was quantitated as described. Briefly male Wistar rats of the age group 5- 6 weeks were maintained on normal rat pellets *ad libidum.* Rats were divided into four groups viz. Groups I, II, III and IV.

Group I : Untreated
Group II : Animals treated with Doxorubicin
Group III : Animals treated with Doxorubicin and 5- Methoxytryptamine
Group IV : Animals treated with 5- Methoxytryptamine.

Each group consisted of 6 animals. 5 mg /kg of Doxorubicinwas administered intraperitoneally to animals of groups II and III as two equal divided doses once every seven days. The animals comprising Group III were injected intraperitoneally with 17.5 mg/kg of 5- Methoxytryptamine, as seven equal doses given every alternate day. The animals of the group III were injected with 5- Methoxytryptamine, 30 minutes prior to the Doxorubicin injection. The animals comprising Group IV were treated with 17.5 mg/kg of 5-Methoxytryptamine as seven equal doses, given every alternate day. At the 41$^{st}$ day of the study, blood was collected from the retroorbital vein of all the animals in the study. The blood was kept at room temperature for 15 minutes, and spun at 3000 rpm for 15 minutes. The serum was separated and immediately estimated for the levels of CK - MB. The quantitation was carried out by kits procured from Bayer Diagnostics using RA 50 Chemistry Analyzer (Bayer Diagnostics), as per the manufacturers instructions.

As shown in Table 5, treatment with 5- Methoxytryptamine reduces the serum levels of CK-MB in Doxorubicin treated animals, which is indicative of its efficacy in reducing / lowering the injury /ischemia/stress/hypoxia caused to the cardiac tissue by Adriamycin treatment.

Table 5 :

| Serum CK - MB levels in Doxorubicin treated animals in a subchronic study. | |
|---|---|
| GROUP | CK - MB (U/L) |
| I (Untreated) | 382 ± 49 |
| II (Doxorubicin treated) | 658 ± 19 |
| III (Doxorubicin and 5- Methoxy tryptamine treated) | 432 ± 38 |
| IV (5 Methoxytryptamine treated) | 379 ± 26 |

**Example 6**

Effect of 5 -Methoxytryptamine on circulating levels of Creatine Kinase - MB (CK- MB) in Doxorubicin treated animals in an acute study.

The effect of 5- Methoxy tryptamine on circulating CK - MB levels was quantitated as described. Briefly male Wistar rats of the age group 5- 6 weeks were maintained on normal rat pellets *ad libidum.* Rats were divided into six groups viz. Groups I, II, III, IV, V, VI.

Group I : Untreated
Group II : Animals treated with Doxorubicin (20mg/kg)
Group III : Animals treated with 5- Methoxytryptamine (8.75mg/kg)
Group IV : Animals treated with 5- Methoxytryptamine.(17.5mg/kg)
Group V : Animals treated with Doxorubicin ( 20mg/kg) & 5 Methoxytryptamine (8.7 mg/kg)
Group VI : animals treated with Doxorubicin ( 20mg/kg) and 5 Methoxytryptamine (17.5 mg/kg)

Each group consisted of 5 animals. 20 mg /kg of Doxorubicin was administered intraperitoneally to animals of groups II , V and VI. The animals comprising Group V were injected intraperitoneally with 8.75 mg/kg of 5- Methoxytryptamine, 30-35 minutes prior to the Doxorubicin injection. The animals of the group VI were injected with 17.5 mg/kg of 5- Methoxytryptamine, 30 minutes prior to the Doxorubicin injection. The animals comprising Group III were treated with 8.75 mg/kg of 5-Methoxytryptamine intraperitoneally. The animals comprising Group IV were treated with 17.5 mg/kg of 5-Methoxytryptamine intraperitoneally .48 hours later blood was collected from the retroorbital vein of all the animals in the study. The blood was kept at room temperature for 15 minutes, and spun at 3000 rpm for 15 minutes. The serum was separated and immediately estimated for the levels of CK - MB. The quantitation was carried out by kits procured from Bayer Diagnostics using RA 50 Chemistry Analyzer (Bayer Diagnostics), as per the manufacturers instructions.

As shown in Table 6, treatment with 5- Methoxytryptamine reduces the serum CK - MB levels in Doxorubicin treated animals in an acute model. This is indicative of its efficacy in reducing/lowering the injury/ischemia/ stress/ hypoxia caused to the cardiac tissue by Doxorubicin treatment in an acute model study.

Table 6 : Serum CK - MB levels in Doxorubicin treated animals in an acute study.

| GROUP | CK - MB (U/L) |
|---|---|
| I (Untreated) | 3046 ± 890. 74 |
| II ( Doxorubicin treated 20mg/kg) | 5296 ± 280.63 |
| III (5- Methoxy tryptamine 8.75mg/kg treated) | 3359 ± 1125.43 |
| IV ((5- Methoxy tryptamine 17.5 mg/kg treated) | 2967 ± 649.55 |
| V Doxorubicin ( 20mg/kg ) and 5 Methoxytryptamine ( 8.7 mg/kg) | 3585 ± 911.74 |
| 1V Doxorubicin( 20mg/kg ) and 5 Methoxytryptamine ( 17:5 mg/kg) | 3207 ± 399.79 |

**Example 6**

Effect of 5-Methoxytryptamine on circulating levels of Lactate Dehydrogenase (LDH) enzyme in Doxorubicin treated animals.

The effect of 5- Methoxytryptamine on circulating LDH levels was quantitated as described. Briefly male Wistar rats of the age group 5- 6 weeks were maintained on normal rat pellets *ad libidum.* Rats were divided into four groups viz. Groups I, II, III and IV.

Group I : Untreated
Group II : Animals treated with Doxorubicin
Group III : Animals treated with Doxorubicin and 5- Methoxytryptamine
Group IV : Animals treated with 5 Methoxytryptamine

Each group consisted of 6 animals. 5 mg /kg of Doxorubicin was administered intraperitoneally to animals of groups II and III as two equal divided doses once every seven days. The animals comprising Group III were injected intraperitoneally with 17.5 mg/kg of 5-Methoxytryptamine, as seven equal doses given every alternate day. The animals

of the group III were injected with 5- Methoxytryptamine, 30 minutes prior to the Doxorubicin injection. The animals comprising Group IV were treated with 17.5 mg/kg of 5- Methoxytryptamine as seven equal doses, given every alternate day. At the 41st day of the study, blood was collected from the retroorbital vein of all the animals in the study. The blood was kept at room temperature for 15 minutes, and spun at 3000rpm for 15 minutes. The serum was separated and immediately estimated for the levels of LDH. The quantitation was carried out by kits procured from Bayer Diagnostics using RA 50 Chemistry Analyzer (Bayer Diagnostics), as per the manufacturer's instructions.

As shown in Table 6, treatment with 5- Methoxytryptamine reduces the LDH levels in Doxorubicin treated animals.

Table 6 : LDH levels in Doxorubicin treated animals.

| GROUP | LDH ( U/L) |
|---|---|
| I (Untreated) | 1018 ± 169 |
| II (Doxorubicin treated) | 1296 ± 83 |
| III (Doxorubicin & 5 Methoxy tryptamine treated). | 1005 ± 38 |
| IV (5 -Methoxy tryptamine treated) | 1009 ± 26 |

## Example 7

**Effect of addition of 5-Methoxy tryptamine on anticancer activity of Doxorubicin *in vitro.***

Experiments were conducted to study the effect of addition of 5-Methoxytryptamine on the anticancer activities of Doxorubicin *in vitro* in human tumour cell lines by performing the MTT cytotoxicity assay (Mosmann, T., J. Immunological Methods, 65:55; 1983). These cell lines included MiaPaCa2 (pancreatic cancer), DU145 ( Prostate cancer), Breast (MCF7) and colon cancer (HT 29). Briefly, 10000 cells of the cultured human tumor cells were separately seeded per well in a 96-well culture plate and incubated with 5-Methoxytryptamine or Doxorubicin or co-incubated with 5-Methoxy Tryptamine and Doxorubicin. 5- Methoxytryptamine was dissolved in 3.5% ethanol in saline. Doxorubicin was dissolved in saline immediately before use. The cells in the control experiments were treated with the appropriate concentrations of the vehicles .The concentration of 5 Methoxytryptamine or Doxorubicin varied from 1 ng/ml- 1000 ng/ml. The effect of co- incubation of 5 Methoxytryptamine and Doxorubicin on the cytotoxicity of Doxorubicin were carried out at the $ED_{50}$ or $ED_{100}$ concentrations of Doxorubicin co - incubated with 5 Methoxytryptamine at a concentration of 1 ug/ ml. All experiments were carried out in triplicates at 37°C in a $CO_2$ incubator. After 72 hours, the assay was terminated and percent cytoxicities and its $IC_{50}$ values calculated. Table 5 below shows that the $IC_{50}$ values of the cytotoxicity of Doxorubicin is not altered by the addition of the 5-Methoxytryptamine at its highest concentration viz. lug/ml.

**Table 5**

| **Effect of co- incubation of 5-Methoxytryptamine and Doxorubicin on the cytotoxicity of Adriamycin in vitro.** | | | |
|---|---|---|---|
| **S.No** | **Cell Line** | **IC50 (Cytotoxicity ng/ml))** | |
| | | Doxorubicin | Doxorubicin & 5MT |
| 1 | MCF 7 ( Breast cancer) | 570 ± 4.5 | 571 ± 5.0 |
| 2 | DU145 (Prostate) | 99 ± 4.0 | 97 ± 4.0 |
| 3 | MiaPaca2 (Pancreatic) | 160 ± 5.0 | 160 ± 5.5 |
| 4 | HT29 (Colon) | 60 ± 5.0 | 60 ± 4.5 |

## Example 6

**Preparation of Syrup of 5-Methoxytryptamine**

1.0gm of 5-Methoxytryptamine was dissolved in 5ml of alcohol and added to 45 ml of sugar syrup already containing sufficient amounts of buffers, approved color, flavour and other stabilizers.

**Example 7**

**Preparation of Hard Gelatin Capsules of 5-Methoxytryptamine**

According to the batch size required suitable amount of 5-Methoxytryptamine was mixed with excipients like lubricants and glidants exemplified by but not limited to talc, magnesium stearate, colloidal silica, etc. and filled into hard gelatin capsules.

**Example - 8**

**Preparation of Injection of 5-Methoxytryptamine**

100 mg of 5-Methoxytryptamine was dissolved in 0.3 ml of ethanol and made up the volume to one ml with surfactants exemplified by but not limited to polysorbates like polysorbate 80, polysorbate 60, polysorbate 20 etc, Cremophor ELP. Alternatively, the compostion may contain cosolvents .like PEG 300, Glycerol, Propylene glycol etc.

## Claims

1. Use of 5-methoxy tryptamine or a salt thereof for the manufacture of a medicament for the prevention and/or treatment of doxorubicin induced cardiotoxicity in a subject.

2. Use as claimed in claim 1, wherein the amount of 5-methoxy tryptamine or its salt in a unit dose of said medicament is in the range of 5 to 500 mg.

3. Use as claimed in any one of claims 1 and 2, wherein the doxorubicin induced cardiotoxicity is in the form of myocardial tissue injury.

4. Use of a pharmaceutical composition of 5-methoxy tryptamine for the manufacture of a medicament for the prevention and/or treatment of doxorubicin induced cardiotoxicity in a subject.

## Patentansprüche

1. Verwendung von 5-Methoxytryptamin oder eines Salzes davon zur Herstellung eines Medikaments zur Vorbeugung und/oder Behandlung von durch Doxorubicin herbeigeführter Herzschädigung bei einem Patiente n.

2. Verwendung nach Anspruch 1, wobei die Menge an 5-Methoxytryptamin oder dessen Salzes in einer Dosiseinheit des Medikaments im Bereich von 5 bis 500 mg liegt.

3. Verwendung nach einem der Ansprüche 1 und 2, wobei die durch Doxorubicin herbeigeführte Herzschädigung in Form einer Herzmuskelgewebeverletzung vorliegt.

4. Verwendung eines Arzneimittels aus 5-Methoxytryptamin zur Herstellung eines Medikaments zur Vorbeugung und/oder Behandlung von durch Doxorubicin herbeigeführter Herzschädigung bei einem Patienten.

## Revendications

1. Utilisation de 5-méthoxytryptamine ou d'un sel de celle-ci pour la préparation d'un médicament pour la prévention et/ou le traitement d'une cardiotoxicité induite par la doxorubicine chez un sujet.

2. Utilisation selon la revendication 1, dans laquelle la quantité de 5-méthoxytryptamine ou de son sel dans une dose unitaire dudit médicament se situe dans la plage de 5 à 500 mg.

3. Utilisation selon l'une quelconque des revendications 1 et 2, dans laquelle la cardiotoxicité induite par la doxorubicine est présente sous la forme d'une lésion du tissu myocardique.

4. Utilisation d'une composition pharmaceutique de 5-méthoxytryptamine pour la préparation d'un médicament pour la prévention et/ou le traitement d'une cardiotoxicité induite par la doxorubicine chez un sujet.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BURKHARD ; POEGGELER et al.** *J. Pineal. Res.,* 2002, vol. 33, 20-30
- **KUNA, P.** *Radiobiologia, Radiotherapia,* 1983, vol. 24 (3), 365-76
- **ROZHDESTVENSKII, L. M. ; GROZDOV S.P.** *Radiobiologiya,* 1979, vol. 19 (6), 868-75
- **PARZYCK, DC. et al.** *Radiochemical and Padioanalytical Letters,* 1974, vol. 17 (5-6), 351-358
- **STREFFER, C. ; FLUEGEL, M.** *Strahlentherapie,* 1973, vol. 146 (4), 444-449
- **FEHER, IMRE et al.** *Int. J. Radiat. Biol,* 1968, vol. 14 (3), 257-262
- **SACCO, S. et al.** *Eur. J. Pharmacol.,* 1998, vol. 34, 249-255
- **SZE, S.F. et al.** *J. Neural. Transm. Gen. Sect.,* 1993, vol. 94, 115-126
- **NG, T.B.** *J. Neural Transmission,* 2000, vol. 107 (11
- **CHAN, T.Y. ; TANG, P.L.** *J. Pineal. Res.,* 1993, vol. 14, 27-33
- **PAOLO LISSONI et al.** *Neuroendocrinol Letts,* 2000, vol. 21, 319-323
- **WEISS, R.B.** *Semin. Oncol.,* 1992, vol. 19, 670-686
- **LEFRAK et al.** *Cancer,* 1973, vol. 32, 302-314
- **BORDONI, A. et al.** *Biochim. Biophys. Acta,* 1999, vol. 1440, 100-106
- **YIN, X.** *Biochem. Pharmacol.,* 1998, vol. 56, 87-93
- **HERSHKO, C. et al.** *Leuk. Lymphoma,* 1993, vol. 11, 207-214
- **CINI NERI, G. et al.** *Oncology,* 1991, vol. 48, 327-333
- **THOMAS, C.E. ; AUST, S.D.** *Arch. Biochem. Biophys.,* 1986, vol. 248, 684-689
- **YOUNG, R.C.** *N. Engl. J. Med.,* 1981, vol. 305, 139-153
- **AROLA, O.J. et al.** *Cancer Res.,* 01 April 2000, vol. 60 (7), 1789-1792
- **M A ABD EL - AZIZ et al.** *Journal of applied Toxicology,* 2001, vol. 21, 469-473
- **XIAOPING LUO et al.** *Biochimica et Biophysica Acta,* 1997, vol. 1360, 45-52
- **YOUNG, RC. et al.** *N. Engl. J. Med.,* 1981, vol. 305, 139-153
- **CARONI, P. et al.** *FEBS Lett,* 1981, vol. 130, 184-186
- **BACHMANN, E. et al.** *Agents Action,* 1975, vol. 5, 383-393
- **KANDA, T. et al.** *Eur. Heart J.,* 1994, vol. 15, 686-690
- **MORGAN, H.E.** *Circulation,* 1993, vol. 87, IV4-IV6
- **BUJA et al.** *Cancer,* 1973, vol. 32, 771-778
- **ARENA, E. et al.** *Int. Res. Commun. Syst. Med. Sci.,* 1974, vol. 2, 1053-1061
- **BRISTOW, M.R. et al.** *Cardiovasc. Pharmacol.,* 1980, vol. 2, 487-515
- **SWAIN, S.M. et al.** *J. Clin. Oncol.,* 1997, vol. 15, 1333-1340
- **SWAIN, S.M.** *J. Clin. Oncol.,* 1997, vol. 15, 1318-1332
- **REITER RUSSEL J et al.** Melatonin: Reducing the toxicity and increasing the efficacy of drugs. *Journal of Pharmacy and Pharmacology,* 10 October 2002, vol. 54, 1299-1321
- **FELESZKO, W. et al.** *Clin. Cancer. Res.,* May 2000, vol. 6, 2044-2052
- **LI, T ; SINGAL, P.** *Circulation,* 2000, vol. 102, 2105-2110
- **JAHNUKAINEN, K. et al.** *Cancer Research,* 01 September 2001, vol. 61, 6423-6427
- **YAMANAKA,S. et al.** *J. Am. Coll. Cardiol.,* 05 March 2003, vol. 41 (5), 570-878
- **SANTOS, D.L. et al.** *Toxicol. Appl. Pharmacol,* 15 December 2002, vol. 185 (3), 218-227
- **EL AZIZ, M. A. et al.** *J. Appl. Toxicol,* 2001, vol. 21, 469-473
- **VENKATESAN, N.** *Br. J. Pharmacol.,* 12 June 1998, vol. 4 (3), 425-427
- **HALLIWELL, B.** *Lancet,* 1994, vol. 344, 721-724
- **CRAPO, J.D. ; TIERNEY, D.F.** *Am. J. Physiol.,* 1974, vol. 226, 1401-1407
- **OBERLEY, L.W. et al.** *Arch. Biochem. Biophys.,* 1987, vol. 54, 69-80
- **WARNER, B.B. et al.** *Am. J. Physiol.,* 1996, vol. 271, L 150-L158
- **DOWNEY, J.M. et al.** *Free Radic. Res. Commun.,* 1991, vol. 12- 13 (Pt2), 703-720
- **ROBINSON, DAVID J. et al.** *J. of Emergency Medicine,* 1999, vol. 17 (1), 95-104
- **TSUNG, S.** *Clin. Chem.,* 1976, vol. 22, 173
- **XIAOPING LUO et al.** *Biochimica et Biophysica Acta,* 1997, vol. 1360, 45-52
- **GILLUM, R.F. et al.** *Am Heart J,* 1984, vol. 108, 150-158

- **GILLUM, R.F. et al.** *Am. Heart. J.,* 1984, vol. 108, 150-158
- **M A ABD EL - AZIZ et al.** *ournal of applied Toxicology,* 2001, vol. 21, 469-473
- **SHERIF Y. SAAD.** *Pharmaceutical Research,* 2001, vol. 43 (3), 211-217
- **TAI K. YEUNG et al.** *Cancer Chemother Pharmacol,* 1992, vol. 30, 58-64
- **WOLF, P.L.** *Clin. Lab. Med.,* 1989, vol. 9, 655
- **HYCON, LEE et al.** *Arch. Pharm. Res.,* vol. 19 (3), 223-227
- **UCHIYAMA ; MIHARA, M.** *Anal. Biochem.,* 1978, vol. 86, 271-278
- **KAHHAR et al.** *Indian Journal of Biochem. and Biophys.,* April 1984, vol. 21, 130-132
- **UCHIYAMA ; MIHARA, M.** *Anal Biochem.,* 1978, vol. 86, 271-278
- **MOSMANN, T.** *J. Immunological Methods,* 1983, vol. 65, 55